# EUROPEAN PATENT SPECIFICATION

(11) **EP 4 422 497 B1**
(45) Date of publication and mention of the grant of the patent: **29.07.2026**
(21) Application number: 22808695.5
(22) Date of filing: 20.10.2022
(51) Int. Cl.: A61B 5/29, A61B 5/349, A61B 5/07, A61B 5/283, A61B 5/00, A61N 1/375, A61B 5/353, A61B 5/355, A61B 5/366

(54) **IMPLANTABLE MEDICAL DEVICE FOR SENSING PHYSIOLOGICAL SIGNALS**
IMPLANTIERBARE MEDIZINISCHE VORRICHTUNG ZUR ERFASSUNG VON PHYSIOLOGISCHEN SIGNALEN
DISPOSITIF MÉDICAL IMPLANTABLE POUR DÉTECTER DES SIGNAUX PHYSIOLOGIQUES

(30) Priority: 26.10.2021 US 202163271903 P; 09.11.2021 EP 21207258
(43) Date of publication of application: 04.09.2024
(73) Proprietor: BIOTRONIK SE & Co. KG, 12359 Berlin (DE)
(72) Inventor: STARKE, Marcel, 15732 Eichwalde (DE); ROMBERG, Jan, 12347 Berlin (DE); KRAUS, Alexander, 10407 Berlin (DE); REDDY, Ravi Kiran Kondama, Portland, Oregon 97221 (US)
(74) Representative: Biotronik Corporate Services SE
(86) International application number: PCT/EP2022/079221
(87) International publication number: WO 2023/072726

(56) References cited:
- US-A1- 2013 138 006
- US-A1- 2014 114 372
- US-A1- 2016 144 190
- US-A1- 2018 264 270
- US-A1- 2021 000 418
- US-A1- 2021 060 346
- US-A1- 2021 251 551

## Description

The instant invention generally relates to an implantable medical device for sensing physiological signals which is to be implanted into a patient for carrying out a diagnostic and/or therapeutic function.

An implantable medical device of this kind may for example be a pacemaker, an implantable cardioverter defibrillator, a sensor device such as a bio-sensor, or a recording device such as a loop recorder. The implantable medical device herein is configured to sense physiological signals. For example, the implantable medical device may be a recording device which is configured to record physiological signals and to communicate recorded physiological signals or information derived from recorded physiological signals to an external device in the context of a home monitoring system. Physiological signals may be electrophysiological signals, in particular electrocardiogram signals.

An implantable medical device as for example described in EP 3 278 836 B1 may for example comprise a housing and an arrangement of electrode poles arranged on the housing. The electrode poles herein are arranged on the housing of the implantable medical device such that the electrode poles are aligned along a longitudinal axis along which the implantable medical device extends. The electrode poles may for example be formed by housing segments which are made from an electrically conductive material such as a metal material and are exposed to the outside such that they may be brought into electrical contact with surrounding tissue in order to establish an electrical coupling to the tissue in an implanted state of the implantable medical device.

Further implantable medical devices are known from US 2016/0144190 A1, US 2021/060346 A1, US 2014/0114372 A1 and US 2021/0251551 A1.

An implantable medical device as used in a home monitoring system shall allow for a reliable monitoring of a physiological state of a patient. In particular, using the implantable medical device it shall be possible to reliably detect an abnormal cardiac state based on recorded physiological signals. If an abnormality is detected in physiological signals, the implantable medical device shall be enabled to communicate with for example an external device of a home monitoring system in order to trigger a message to a service center to alert medical personnel of a potential need for attention.

It herein is desirable to be able to reliably monitor certain waveform features in physiological signals, for example in electrocardiogram signals, such as a P-wave, a QRS waveform or a T-wave. If the implantable medical device for example is implanted outside of the heart of the patient and does not comprise leads extending into the heart, some waveform features in the electrocardiogram signals may be larger than others, making it possibly difficult to monitor certain waveform features, such as a P-wave in a cardiac cycle stemming from atrial activity.

It is an object of the instant invention to provide an implantable medical device and a method for operating an implantable medical device which in a reliable way allow to sense and process physiological signals to assess a cardiac function based on the physiological signals. This object is achieved by means of an implantable medical device comprising the features of claim 1 and a method of operating an implantable medical device according to claim 12. The dependent claims concern embodiments of the invention.

The scope of the present invention is defined by the appended claims.

In one aspect, an implantable medical device for sensing physiological signals, for example electrocardiogram signals, comprises an arrangement of at least a first electrode pole, a second electrode pole and a third electrode pole, said arrangement of at least the first electrode pole, the second electrode pole and the third electrode pole being configured to sense physiological signals. The implantable medical device further comprises a processing module for processing signals received via said arrangement of at least the first electrode pole, the second electrode pole and the third electrode pole. Herein, the processing module is configured to process different physiological signals received by different pairs of electrode poles of the arrangement of at least the first electrode pole, the second electrode and the third electrode pole and to assess a cardiac function based on the processing.

The implantable medical device, accordingly, comprises multiple electrode poles, are arranged at different axial positions on the implantable medical device. "Multiple" in this context means "at least three". By means of the different electrode poles an electrical coupling to surrounding tissue is established when the implantable medical device is implanted in a patient, such that physiological signals may be sensed using the different electrode poles.

Physiological signals herein are sensed using different pairs of electrode poles to obtain different physiological signals, which may be processed by the processing module in order to assess a cardiac function. If an abnormality based on a monitoring of a particular cardiac function is detected, this may be interpreted as a change in a physiological state of the patient, and accordingly for example a message to an external device may be transmitted to trigger an alert in a home monitoring system.

As the cardiac function is assessed based on the reception of different physiological signals which are received by different pairs of different electrode poles of the implantable medical device, a diagnostic precision may be improved. As the different physiological signals are received by different pairs of the different electrode poles and as the spatial reception characteristics of the different pairs of the electrode poles differ, a particular waveform feature, such as a P-wave feature, may be enhanced in one electrocardiogram signal in comparison to another electrocardiogram signal.

In one embodiment, the arrangement of multiple electrode poles, the arrangement of electrode poles or the arrangement of at least the first electrode pole, the second electrode pole and the third electrode pole (three different terms for the same arrangement) contains more than three (four or more) electrode poles. Thus, the mentioned diagnostic precision may be further improved.

In one embodiment, the electrode poles are arranged aligned along a longitudinal axis. If the electrode poles are aligned along the longitudinal axis of the implantable medical device, different pairs of electrode poles span different reception vectors. One reception vector herein may be closer to a particular cardiac region than another reception vector, such that a particular pair of electrode poles may be more suitable to detect and to monitor a particular waveform feature than another pair of electrode poles.

For example, the implantable medical device may be a monitoring device which is to be implanted in a location external to the heart.

In one embodiment, the processing module is configured to identify a waveform feature in at least one of the different physiological signals, in particular different electrocardiogram signals. The waveform feature may for example be a P-wave, QRS-wave or T-wave feature. All of these have specific frequency components and can be better displayed with separately adjustable amplification channels e.g. with adapted filter characteristics or gain levels or by switching settings for a focus function e.g. for a differential analysis of ECG or other electrical physiological signals.

If the implantable medical device is for example implanted along a substantially vertical direction (with respect to a height direction - from head to feet - of the patient), one pair of electrode poles may come to lie closer for example to the right atrium of the heart than another pair of electrode poles. If a certain waveform feature, for example the P-wave, is to be monitored, a pair of electrode poles closer to the atrium may allow for a stronger reception of signals originating from the atrium, such that by means of the pair of electrode poles coming to rest closest to the atrium an enhanced P-wave feature detection may be achieved.

If the implantable medical device is for example implanted along a substantially transverse direction - transverse to a height direction of the patient - into the patient, one pair of electrode poles may come to lie closer to the right ventricle, whereas another pair of electrode poles comes to lie closer to the left ventricle. Making use of the different pairs of electrode poles, hence, physiological signals may be received which predominantly originate from the right ventricle or from the left ventricle, making it possible to assess a synchronicity/asynchronicity between a right ventricular chamber polarization and a left ventricular chamber polarization.

Generally, the cardiac action potential is understood as a brief change in voltage (membrane potential) across the cell membrane of heart cells. This is caused by the movement of charged atoms (called ions) between the inside and outside of the cell, through proteins called ion channels. Within cardiac activity action potentials begin with a voltage becoming more positive, known as depolarization, followed by a phase in which the action potentials return to negative, known as repolarization. Action potential activity is recorded as physiological signals, wherein based on the different physiological signals action potential activity for e.g. the right ventricle and the left ventricle may be monitored. Based on the monitoring a potential asynchronicity between the right ventricular activity and the left ventricular activity can be assessed.

In yet another embodiment, the processing module may be configured to assess a dislocation of the implantable medical device by monitoring at least one waveform feature in at least one of the different physiological signals. By monitoring one or multiple waveform features, such as for example a P-wave feature in relation to a QRS waveform or a T-wave, in one or multiple of the different physiological signals it may be assessed whether the implantable medical device, in its implanted state, has changed its location or orientation, which may come with a change in timing, level and/or length of a waveform feature over the course of cardiac cycles in one or multiple physiological signals.

Reliable P wave detection may be necessary for accurate and automatic analysis of electrocardiogram (ECG) signals or other physiological signals. Under normal or usual conditions, P-wave detection in physiological conditions is understood with reasonable methods. However, methods for P-wave detection during various cardiovascular pathologies may be ill-defined and the performance could be insufficient. This work introduces a novel method, based on a differential signal analysis combined with digital filtering, as well as innovative rules that improve P-wave detection. Current P-wave detection methodologies are based on identification of R waves and identification of P-waves in the vicinity, however, for various AV dissociation pathologies such as second- and third-degree AV block, as well as some types of supraventricular tachycardia with AV dissociations, where the normal time ordering of the cardiac signals is not synchronized these approaches are not sufficient.

In the device configuration with at least two sets of electrodes, the waveform recorded from each set of electrodes can be treated independently. Delayed and asynchronous activation of the left atrium (LA) is a precursor for the development of atrial fibrillation (AF) and can be assessed quantitatively by computing the P-wave duration (PWD) using the current design proposed here. Additional indices such as P-wave dispersion, voltage, and morphology, and the ECG presence of interatrial block can be measured using the approach proposed here. Amplification of the ECG measured as a difference between the two measured signals provides an approach to detect the earliest deflection from the isoelectric line, the earliest point of the P-wave and accurately identify the parameters listed above.

In one embodiment, the processing module is configured to determine at least one of a P-wave timing, a P-wave level, a P-wave length, a QRS waveform timing, a QRS waveform level, a QRS waveform length, a T-wave timing, a T-wave level, and a T-wave length in at least one of the different physiological signals. Based on characteristic parameters as identified in association with a certain waveform feature, such as a P-wave, a QRS waveform or a T-wave, a monitoring may be established, for example for monitoring a P-wave with enhanced sensitivity using the implantable medical device. In particular, the processing module may be configured to process a first physiological signal received by a first pair of electrode poles in a first processing channel and a second physiological signal received by a second pair of electrode poles in a second processing channel, wherein further physiological signals received by other pairs of electrode poles may be processed in further processing channels. Processing of the different physiological signals hence may take place in different channels, wherein within each channel for example an amplification, a filtering and/or an analog-to-digital conversion may take place.

The channels may each be amplified and/or filtered separately, for example with filters and amplifiers that are each matched or adjusted to the physiological signals. In addition or as an alternative, different signal processing, e.g. a Fourier analysis, may be carried out for each channel, e.g. for a diagnosis. For example, if a P-wave feature shall be monitored by using a processing channel allowing for an enhanced reception of the P-wave feature, such processing channel may be selected out of the multiple processing channels in which the P-wave feature is received with the strongest amplitude. In case a P-wave feature in the particular, selected processing channel is lost, it may be switched to a different channel, such that a continuous monitoring of for example the P-wave over a sequence of cardiac cycles becomes possible based on a selection of a suitable processing channel out of the multiple different processing channels.

The processing of the different signals herein beneficially takes place synchronously, such that the different physiological signals received via the different pairs of electrode poles are processed synchronously to for example assess and monitor waveform features in the different physiological signals.

In one embodiment, the first electrode pole and the second electrode pole define a first signal reception vector therebetween pointing along the longitudinal axis, the second electrode pole and the third electrode pole define a second signal reception vector therebetween pointing along the longitudinal axis, and the third electrode pole and the first electrode pole define a third signal reception vector therebetween pointing along the longitudinal axis. Because the electrode poles are aligned along the longitudinal axis, the signal reception vectors are pointing in parallel to one another, but due to the different axial locations of the different electrode poles differ from one another. One pair of electrode poles, for example the first electrode pole and the second electrode pole, hence senses a different signal than another pair of electrode poles, for example the first electrode pole and the third electrode pole.

In one embodiment, the implantable medical device comprises a housing, wherein the first electrode pole is arranged at a first end of the housing, the second electrode pole is arranged at a second end of the housing, and the third electrode pole is arranged at a location in between said first end and said second end. The housing may for example have a rigid shape, wherein the electrode poles may be formed by certain housing segments, or may be formed by discrete pole elements arranged on the housing. For example, the housing may comprise a first housing segment in which the processing module is received, a second housing segment in which a battery module is received, and a third housing segment for example formed by a so-called header portion extending, for example longitudinally, from another housing segment.

In particular in case the first electrode pole is arranged on a first end of the housing and the second electrode pole is arranged at a second end of the housing opposite to the first end, a first pair of electrode poles may beneficially be formed by the first electrode pole and the second electrode pole. The first electrode pole and the second electrode pole in this case may form a comparatively long signal reception vector, hence allowing for a reception of comparatively strong physiological signals from regions remote to the implantable medical device. A second pair of electrode poles may be formed by the first and the third or the second and the third electrode pole, defining a signal reception vector which is shorter than the first signal reception vector in between the first electrode pole and the second electrode pole.

The implantable medical device with its housing in particular may be configured for implantation external to the heart of the patient, for example subcutaneously within the patient. The electrodes herein are arranged on the housing. The implantable medical device for example does not comprise leads which extend into the heart, an implantable medical device of this kind also being denoted as non-transvenous implantable medical device.

In one embodiment, the processing module is configured to assess a cardiac function based on a comparison of at least two of the different physiological signals. For example, a particular waveform feature such as a P-wave may be identified in different physiological signals, in particular in different electrocardiogram signals and may be compared in order to select a processing channel allowing for an enhanced monitoring of the waveform feature. In one embodiment, the processing module is configured to assess a cardiac function by feeding at least two of the different physiological signals to a combiner and by processing a combined signal output by the combiner. Using the combiner for example a signal summation (signal A + signal B), a signal difference (signal A - signal B) or a signal relation (signal A / signal B) may be formed. Based on the combined signal a waveform feature may be assessed, for example to enhance a particular feature such as a P-wave feature by forming a subtraction of different signals.

If the implantable medical device is used in a home monitoring system, the implantable medical device may comprise communication circuitry configured to establish a communication to an external device which is external to the patient. In case an abnormality is identified based on the processing of the different physiological signals, the processing module may for example generate and transmit a warning message to the external device, such that the external device is informed about a potentially critical change in condition.

In another aspect, a method for operating an implantable medical device for sensing physiological signals as defined in claim 12 is provided.

The advantages and advantageous embodiments described above for the implantable medical device equally apply to the method, such that it shall be referred to the above in this respect.

The various features and advantages of the present invention may be more readily understood with reference to the following detailed description and the embodiments shown in the drawings. Herein,
- Fig. 1: shows a schematic drawing of an implantable medical device implanted in a patient;
- Fig. 2: shows a schematic drawing of an embodiment of an implantable medical device comprising an arrangement of electrode poles;
- Fig. 3: shows a schematic drawing of another embodiment of an implantable medical device;
- Fig. 4: shows a schematic drawing of an implantable medical device, illustrating a signal reception using a pair of electrode poles;
- Fig. 5: shows a drawing of an implantable medical device, illustrating a signal reception using different signal vectors formed by different pairs of electrode poles;
- Fig. 6: shows a schematic drawing of a processing of received signals;
- Fig. 7: shows a schematic drawing of another embodiment of a processing of received signals;
- Fig. 8: shows a schematic drawing of a processing module comprising multiple processing channels;
- Fig. 9: shows an example of an implantable medical device comprising a multiplicity of electrode poles arranged on a housing;
- Fig. 10: shows a multiplicity of exemplary physiological signals received via a multiplicity of processing channels of an implantable medical device;
- Fig. 11: shows a schematic representation of a cardiac cycle of an physiological signal;
- Fig. 12: shows an embodiment of an implantable medical device in a first orientation with respect to a heart;
- Fig. 13: shows an embodiment of an implantable medical device in another, second orientation with respect to the heart; and
- Fig. 14: shows an embodiment of an implantable medical device in relation to a patient's heart, in case of a dislocation of the implantable medical device.

Subsequently, embodiments shall be described in detail with reference to the drawings for better understanding the present invention which is defined by the appended claims. In the drawings, like reference numerals designate like structural elements.

It is to be noted that the embodiments are not limiting for the invention, but merely represent illustrative examples.

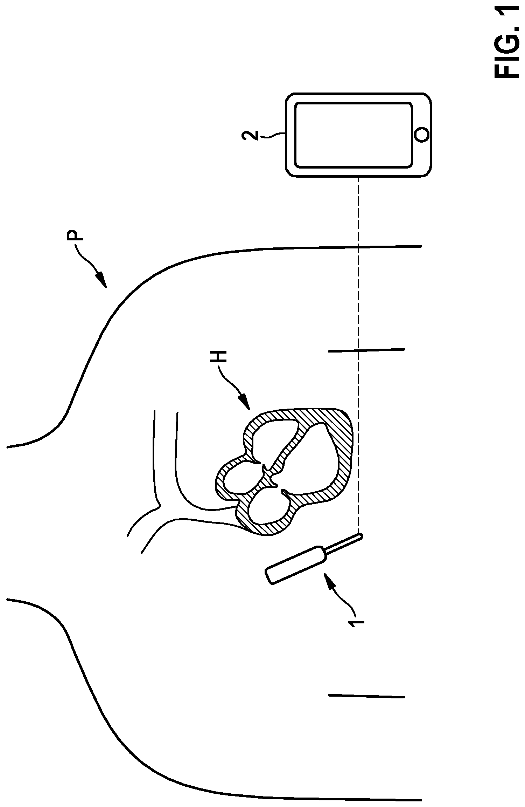

Referring to Fig. 1, in one embodiment an implantable medical device 1 is implanted (for example subcutaneously) into a patient P for serving a therapeutic and/or diagnostic function. The implantable medical device 1 may for example be implanted subcutaneously into the patient P for monitoring cardiac activity of the patient's heart H. The implantable medical device 1, for this, comprises an arrangement of electrode poles which are used to couple to surrounding tissue and to sense physiological signals, here in particular electrocardiogram signals, originating from the heart H.

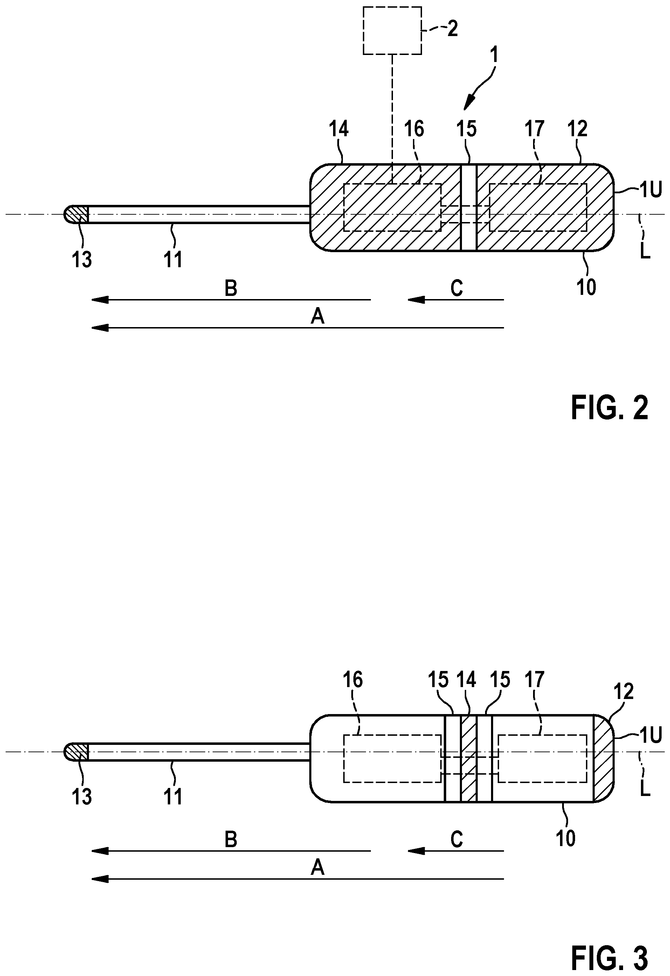

Referring now to Fig. 2, in one embodiment the implantable medical device 1 comprises a housing 10 formed by different housing segments, the housing 10 enclosing and encapsulating a processing module 16 formed by electronic circuitry and a battery module 17. In particular, a first housing segment may receive and enclose the processing module 16, whereas a second housing segment receives and encloses the battery module 17. Another housing segment 11 longitudinally extends from the first and second housing segments and forms a header portion having reduced cross-sectional dimensions with respect to the other housing segments.

In the embodiment of Fig. 2, a first electrode pole 12 is formed by the housing segment enclosing the battery module 17, a second electrode pole 13 is arranged at a far end of the housing segment 11 forming the header portion, and a third electrode pole 14 is formed by the housing segment enclosing the processing module 16. The implantable medical device 1 with its housing 10 generally extends along a longitudinal axis L, the electrode poles 12, 13, 14 being aligned along the longitudinal axis L and being axially displaced with respect to one another along the longitudinal axis L. The electrode poles 12, 13, 14 herein are electrically separated from one another, an electrically insulating segment 15 being arranged in between the electrode poles 12, 14 formed on the main housing portion and the header portion formed by the housing segment 11 separating the electrode pole 13 from the other two electrode poles 12, 14.

At this point it should be explicitly pointed out that an alignment of the electrode poles 12, 13, 14, any housing segments and/or other elements along the longitudinal axis L is not absolutely necessary. Other arrangements are also possible. For example, the electrode poles 12, 13, 14 and/or further electrode poles may be arranged alternately on a top and a bottom, or on a left and a right side of the implantable medical device.

In the embodiment of Fig. 2, the electrode poles 12, 13, 14 may be formed by portions of the housing 10 itself, the housing 10 being made for example from an electrically conductive material, in particular a metal material. By exposing portions of the housing 10 towards the outside, the electrode poles 12, 13, 14 are formed and may electrically contact with surrounding tissue in order to establish a coupling between the electrode poles 12, 13, 14 to the surrounding tissue.

Referring now to Fig. 3, in another embodiment the first electrode pole 12 is formed at an end of a housing segment of the housing 10 encapsulating the battery module 17, whereas the electrode pole 14 is formed by an electrode element which is electrically insulated from other portions of the housing 10 by electrically insulating segments 15. For example, a multilayered pole element may be employed for forming the electrode pole 14, as it is described for example in EP 3 278 836 B1. The electrode pole 13 again is formed at a far end of the housing segment 11 forming the header portion.

In any of the embodiments of Fig. 2 and 3, using the arrangement of electrode poles 12, 13, 14, physiological signals may be received and processed by the processing module 16. Based on the processing, a communication with an external device 2 may be established, for example to transmit alert messages to the external device 2 for example within the context of a home monitoring system for monitoring a physiological state of the patient P.

The different electrode poles 12, 13, 14 herein define signal reception vectors A, B, C by means of which physiological signals may be received using pairs of associated electrode poles 12, 13, 14. In particular, a first signal reception vector A is formed between the first electrode pole 12 and the second electrode pole 13, a second signal reception vector B is formed between the third electrode pole 14 and the first second electrode pole 13, and a third signal reception vector C is formed between the first electrode pole 12 and the third electrode pole 14. As the first electrode pole 12 and the second electrode pole 13 are arranged at opposite ends of the housing 10, the associated signal reception vector A is longer than the other two signal reception vectors B, C.

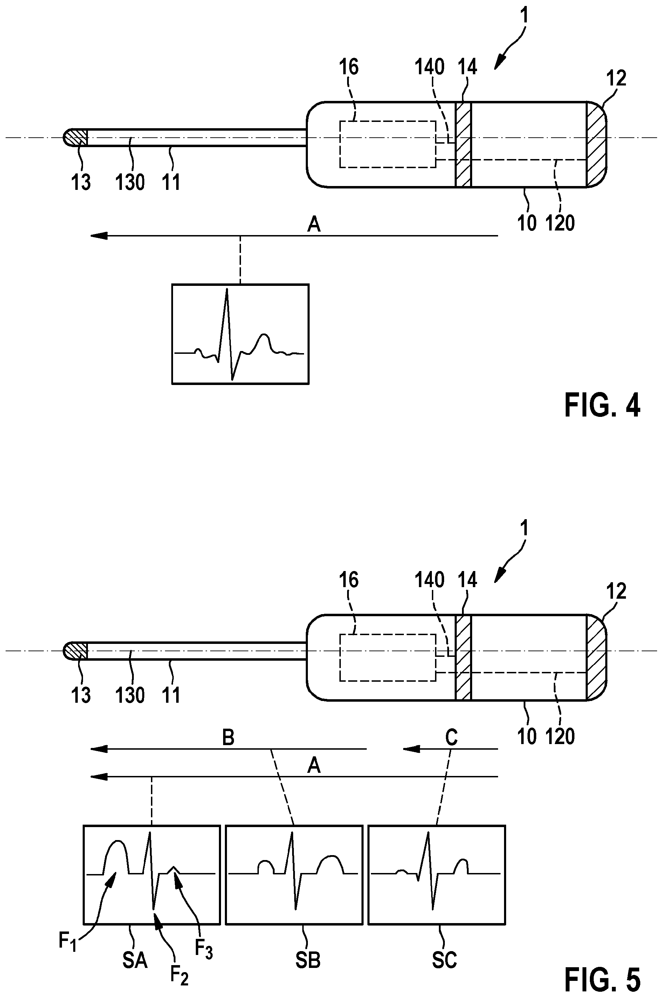

Referring now to Fig. 4, for a regular signal reception for example a pair of electrode poles formed by the first electrode pole 12 and the second electrode pole 13 may be used, defining a signal reception vector A as illustrated in Fig. 4. Using the signal reception vector A physiological signals may be recorded and may be processed within the processing module 16, which is connected to the different electrode poles 12, 13, 14 by means of electrical connection lines 120, 130, 140. Using the signal reception vector A for regular signal reception may come with the benefit of a strong signal reception even from remote regions, as the electrode poles 12, 13 define a comparatively long signal reception vector A for receiving signals from surrounding tissue.

Referring now to Fig. 5, the different electrode poles 12, 13, 14 form different pairs of electrode poles 12, 13, 14 spanning different signal reception vectors A, B, C. By means of the different signal reception vectors A, B, C different physiological signals SA, SB, SC may be received, which due to the different spatial extension of the different vectors A, B, C differ.

As, due to the different axial position of the electrode poles 12, 13, 14 on the housing 10 of the implantable medical device 1, the signal reception vectors A, B, C are located at and across different spatial regions, one signal vector A, B, C may be located closer to or farther remote from a particular cardiac region, such as the right or left atrium or the right or left ventricle. Based on the spatial positioning of the signal reception vectors A, B, C, hence, within one signal a particular waveform feature may be more pronounced with respect to a signal received by another signal reception vector A, B, C.

For example, as illustrated in Fig. 5, a waveform feature F1 relating to a P-wave in a cardiac cycle of the physiological may be more pronounced in one physiological signal SA, SB, SC in comparison to another physiological signal SA, SB, SC. Likewise, a waveform feature F2 relating to a QRS waveform or a waveform feature F3 relating to the T-wave may be more pronounced or may be smaller in one signal in comparison to another.

Based on the different signal vectors A, B, C, different physiological signals SA, SB, SC may be processed by the processing module 16, and based on the processing a cardiac function may be assessed, for example to monitor a P-wave or for assessing a chamber synchronicity.

For the processing, in principle different approaches can be chosen.

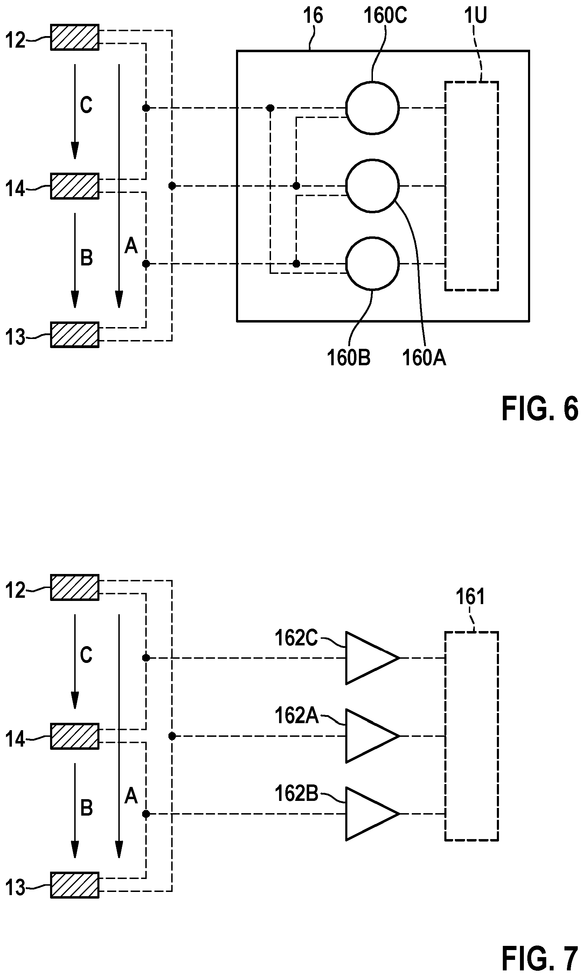

For example, as shown in Fig. 6, signals received via the different signal reception vectors A, B, C may be fed to combiners 160A, 160B, 160C, the combiners 160A, 160B, 160C forming combined signals and forwarding the combined signals to a processing circuitry 161 for a further processing. For example, in the combiners 160A, 160B, 160C a summation of the signals (e.g., signal A + signal B), a difference of the signals (e.g., signal A - signal B), or a relation of the signals (e.g., signal A / signal B) may be formed and may be processed.

In another example, shown in Fig. 7, the different signals sensed via the different signal reception vectors A, B, C may each be fed to an amplifier 162A, 162B, 162C and to a processing circuitry 161.

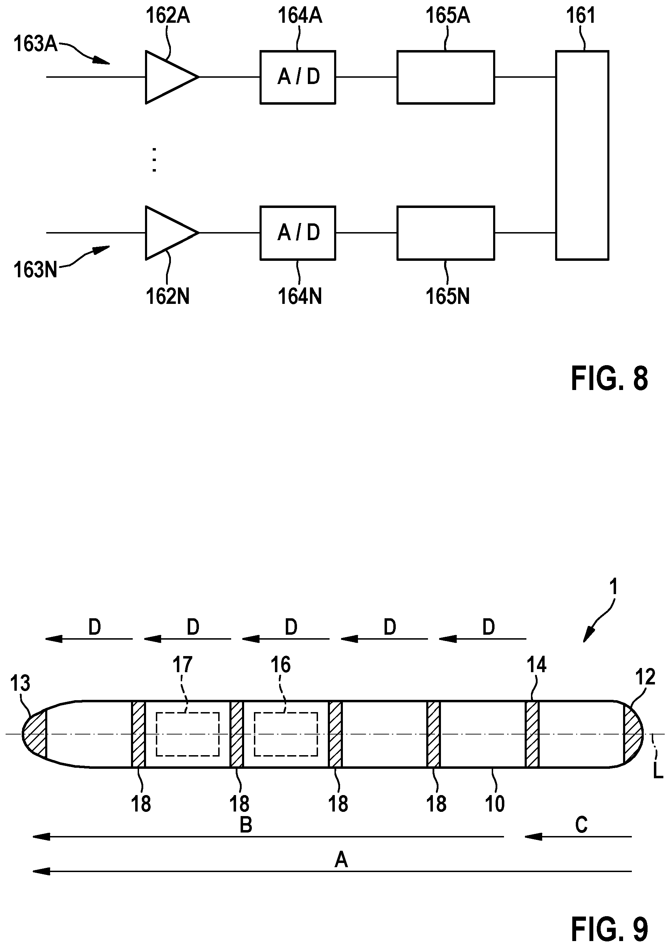

Referring now to Fig. 8, in one example different physiological signals received by different pairs of electrode poles may be processed in different processing channels 163A... 163N. As is schematically illustrated in Fig. 9, in one embodiment the implantable medical device 1 herein may comprise more than three electrode poles 12, 13, 14, 18, wherein the electrode poles 12, 13, 14, 18 may be arranged for example equidistantly or non-equidistantly along the longitudinal direction L on the housing 10 of the implantable medical device 1 such that a multiplicity of different signal reception vectors A, B, C, D are formed between different pairs of electrode poles 12, 13, 14, 18.

Each physiological signal received by a particular pair of electrode poles 12, 13, 14, 18 according to a specific signal reception vector A, B, C, D herein may be processed in a dedicated processing channel 163A... 163N, as illustrated in Fig. 8, wherein each processing channel 163A... 163N may for example comprise an amplifier 162A... 162N, an analog-to-digital converter 164A... 164N, and a filter unit 165A... 165N and may provide an input to a processing circuitry 161 for further processing the different signals of the different processing channels 163A...163N e.g. in the digital domain.

The (analog) amplifier 162A... 162N of each processing channel 163A...163N may comprise a dedicated gain, wherein the gain of the different amplifiers 162A... 162N may differ and may be adapted for enhancement of a particular physiological signal processed in a particular processing channel 163A... 163N.

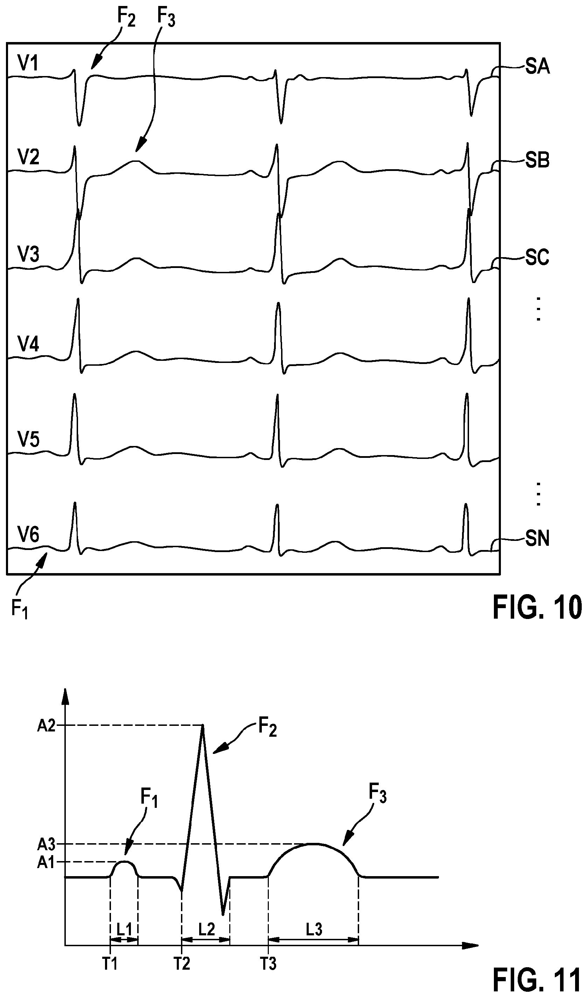

Fig. 10 shows an example of different physiological signals SA, SB, SC... SN received by means of different pairs of electrode poles 12, 13, 14, 18.

Using the different signal reception vectors A, B, C, D, as illustrated in Fig. 9, a spatially selective signal reception may be obtained, allowing for a spatially selective measurement of signals with a spatial resolution corresponding to the distribution of the electrode poles 12, 13, 14, 18 on the housing 10 of the implantable medical device 1. By means of the spatially distributed electrode poles 12, 13, 14, 18 in particular signals may be received differently from different regions of the heart, for example the right or left atrium or the right or left ventricle.

Referring now to Fig. 11, for the processing of the different physiological signals, waveform features F1, F2, F3 relating to a P-wave (waveform feature F1), a QRS waveform (waveform feature F2) and a T-wave (waveform feature F3) may be assessed. For the different waveform features herein parameters such as a timing T1, T2, T3 (for example relating to a start time of the particular waveform feature F1, F2, F3, for example defined by the crossing of a predefined threshold), a level A1, A2, A3 (corresponding to e.g. a maximum amplitude of the specific waveform feature F1, F2, F3) and a length (corresponding to a time length of the specific waveform feature F1, F2, F3) may be determined and assessed.

Within the processing, a comparison may take place, for example by comparing signal levels, lengths or timings in the different physiological signals with respect to one another.

Generally, a processing may take place in one or multiple channels. If for example only one processing channel involving a single signal amplification is used, a signal multiplexing may be employed. The processing may include the use of one or multiple comparators.

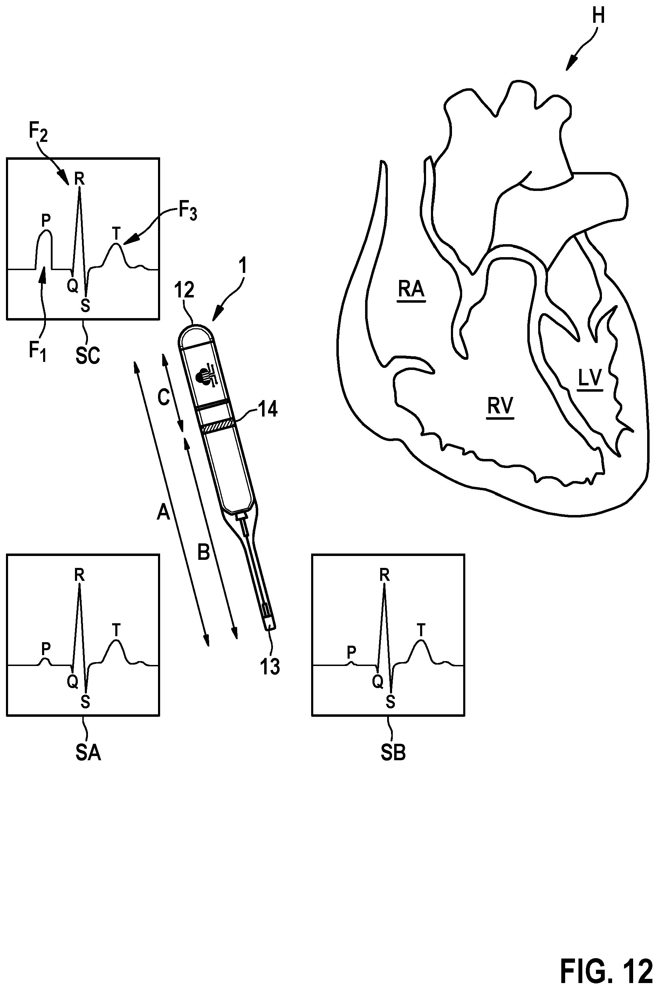

By employing a multichannel processing scheme for processing different physiological signals received by means of different pairs of electrode poles 12, 13, 14, 18, for example an enhanced monitoring of a specific waveform feature F1, F2, F3, for example the P-wave, may be achieved. For example, as illustrated in Fig. 12, in case the implantable medical device 1 is oriented substantially along a perpendicular direction with respect to the heart H, a signal reception vector C spanned between electrode poles 12, 14 may come to lie closest to the right atrium RA, such that due to the spatial proximity of the signal reception vector C to the right atrium RA signals originating from the right atrium RA, namely the P-wave, may be enhanced within the physiological signal SC as received by means of the signal reception vector C, as it is illustrated in Fig. 12. Hence, by selecting the pair of electrode poles 12, 14 and a corresponding processing channel for processing the physiological signal SC received by means of that pair of electrode poles 12, 14, the P-wave (waveform feature F1 in the physiological signal SC) may be monitored in an enhanced fashion.

The enhanced monitoring of the P-wave may be further improved by a suitable processing, such as a selective amplification and/or filtering and/or by e.g. a signal subtraction to form a difference with other received physiological signals SA, SB in order to differentiate the P-wave from other waveform features.

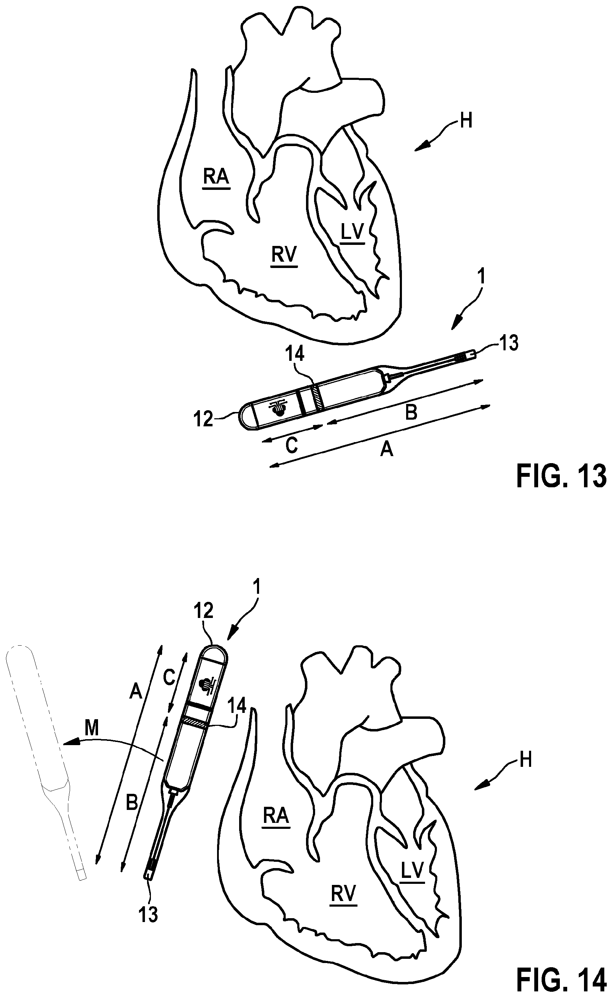

Referring now to Fig. 13, in another example the implantable medical device 1 may be implanted such that it is oriented substantially along a transverse direction, that is transverse to a height direction of the patient. In this case, a pair of electrode poles, namely the pair of electrode poles 12, 14, may lie closer to the right ventricle RV, whereas another pair of electrode poles, namely the pair of electrode poles 13, 14, is closer to the left ventricle LV. In this way signals from the right ventricle RV and from the left ventricle LV may be received and processed in a differentiated fashion, such that for example a synchronicity between cardiac action potential activities in the right ventricle RV and the left ventricle LV may be assessed in order to detect a potential asynchronous chamber polarization.

In yet another example, referring now to Fig. 14, a dislocation of the implantable medical device 1 may be detected by tracking one or multiple particular waveform features F1, F2, F3 in physiological signals received via different signal reception vectors A, B, C. If the implantable medical device 1 in an implanted state is displaced, as illustrated by motion arrow M in Fig. 14, this may give rise to a change of the morphology of the different physiological signals received by means of the different signal reception vectors A, B, C, which may be analyzed in order to identify the change in location/orientation of the implantable medical device 1.

### List of reference numerals

- 1: Implantable medical device
- 10: Housing
- 11: Housing segment
- 12: Electrode pole
- 120: Connection line
- 13: Electrode pole
- 130: Connection line
- 14: Electrode pole
- 140: Connection line
- 15: Electrically insulating segment
- 16: Processing module
- 160A-C: Combiner
- 161: Processing circuitry
- 162A-C: Amplifier
- 163A, 163N: Processing channel
- 164A, 164N: Analog-to-digital converter
- 165A, 165N: Filter unit
- 17: Battery module
- 18: Electrode poles
- 2: External device
- A, B, C, D: Signal reception vector
- A1, A2, A3: Level
- F1, F2, F3: Features
- H: Heart
- L: Longitudinal axis
- L1, L2, L3: Length
- M: Motion arrow
- P: Patient
- SA, SB, SC, SN: Physiological signal
- T1, T2, T3: Timing

## Claims

1. An implantable medical device (1) for sensing physiological signals, comprising:
an arrangement of at least a first electrode pole (12), a second electrode pole (13) and a third electrode pole (14), said arrangement of at least the first electrode pole (12), the second electrode pole (13) and the third electrode pole (14) being configured to sense physiological signals; and
a processing module (16) for processing physiological signals received via said arrangement of at least the first electrode pole (12), the second electrode pole (13) and the third electrode pole (14);
wherein the processing module (16) is configured to process different physiological signals (SA, SB, SC, SN) received by different pairs of electrode poles of the arrangement of at least the first electrode pole (12), the second electrode pole (13) and the third electrode pole (14) and to assess a cardiac function based on the processing;
wherein the processing module (16) is configured to identify a timing relation between a right ventricular action potential activity and a left ventricular action potential activity based on the different physiological signals (SA, SB, SC, SN);
wherein the processing module (16) is configured to process the different physiological signals (SA, SB, SC, SN) in different processing channels (163A, 163N); and
wherein the processing module (16) is configured to select one of the different processing channels (163A, 163N) for monitoring a particular waveform feature (F1-F3) based on an identification of the particular waveform feature (F1-F3) in the different physiological signals (SA, SB, SC, SN).

2. The implantable medical device (1) according to claim 1, wherein the arrangement contains more than three electrode poles (12, 13, 14, 18).

3. The implantable medical device (1) according to claim 1 or 2, wherein the processing module (16) is configured to identify a waveform feature (F1-F3) in at least one of the different physiological signals (SA, SB, SC, SN).

4. The implantable medical device (1) according to claim 3, wherein the waveform feature (F1-F3) is a P-wave feature.

5. The implantable medical device (1) according to one of the preceding claims, wherein the processing module (16) is configured to determine at least one of a P-wave timing (T1), a P-wave level (A1), a P-wave length (L1), a QRS waveform timing (T2), a QRS waveform level (A2), a QRS waveform length (L2), a T-wave timing (T3), a T-wave level (A3), and a T-wave length (L3) in at least one of the different physiological signals (SA, SB, SC, SN).

6. The implantable medical device (1) according to one of the preceding claims, wherein the processing module (16) is configured to perform, in at least one of the different processing channels (163A, 163N) at least one of an amplification, an analog-to-digital conversion and a filtering.

7. The implantable medical device (1) according to one of the preceding claims, wherein the processing module (16) is configured to synchronously process said first signal in said first processing channel and said second signal in said second processing channel.

8. The implantable medical device (1) according to one of the preceding claims, wherein the electrode poles (12, 13, 14) are arranged aligned along a longitudinal axis (L).

9. The implantable medical device (1) according to claim 8, wherein the first electrode pole (12) and the second electrode pole (13) define a first signal reception vector (A) therebetween pointing along the longitudinal axis (L), the second electrode pole (13) and the third electrode pole (14) define a second signal reception vector (B) therebetween pointing along the longitudinal axis (L), and the first electrode pole (12) and the third electrode pole (14) define a third signal reception vector (C) therebetween pointing along the longitudinal axis (L).

10. The implantable medical device (1) according to one of the preceding claims, comprising a housing (10), wherein the first electrode pole (12) is arranged at a first end of the housing (10), the second electrode pole (13) is arranged at a second end of the housing (10) opposite the first end, and the third electrode pole (14) is arranged at a location in between said first end and said second end.

11. The implantable medical device (1) according to one of the preceding claims, wherein the processing module (16) is configured to assess said cardiac function based on a comparison of at least two of the different physiological signals (SA, SB, SC, SN).

12. A method for operating an implantable medical device (1) for sensing physiological signals, comprising:
sensing physiological signals using an arrangement of at least a first electrode pole (12), a second electrode pole (13) and a third electrode pole (14), the first electrode pole (12); and
processing, using a processing module (16), signals received via said arrangement of at least the first electrode pole (12), the second electrode pole (13) and the third electrode pole (14), wherein the processing module (16) processes different physiological signals (SA, SB, SC, SN) received by different pairs of electrode poles of the arrangement of at least the first electrode pole (12), the second electrode pole (13) and the third electrode pole (14) and assesses a cardiac function based on the processing;
wherein the processing module (16) identifies a timing relation between a right ventricular action potential activity and a left ventricular action potential activity based on the different physiological signals (SA, SB, SC, SN);
wherein the processing module (16) processes the different physiological signals (SA, SB, SC, SN) in different processing channels (163A, 163N); and
wherein the processing module (16) selects one of the different processing channels (163A, 163N) for monitoring a particular waveform feature (F1-F3) based on an identification of the particular waveform feature (F1-F3) in the different physiological signals (SA, SB, SC, SN).

## Patentansprüche

1. Ein implantierbares medizinisches Gerät (1) zum Erfassen physiologischer Signale, umfassend:
eine Anordnung aus mindestens einem ersten Elektrodenpol (12), einem zweiten Elektrodenpol (13) und einem dritten Elektrodenpol (14), wobei die Anordnung aus mindestens dem ersten Elektrodenpol (12), dem zweiten Elektrodenpol (13) und dem dritten Elektrodenpol (14) zur Erfassung physiologischer Signale konfiguriert ist; und ein Verarbeitungsmodul (16) zum Verarbeiten physiologischer Signale, die über die Anordnung aus mindestens dem ersten Elektrodenpol (12), dem zweiten Elektrodenpol (13) und dem dritten Elektrodenpol (14) empfangen werden;
wobei das Verarbeitungsmodul (16) so konfiguriert ist, dass es verschiedene physiologische Signale (SA, SB, SC, SN), die von verschiedenen Paaren von Elektrodenpolen der Anordnung aus mindestens dem ersten Elektrodenpol (12), dem zweiten Elektrodenpol (13) und dem dritten Elektrodenpol (14) empfangen werden, verarbeitet und auf der Grundlage der Verarbeitung eine Herzfunktion bewertet;
wobei das Verarbeitungsmodul (16) so konfiguriert ist, dass es auf der Grundlage der verschiedenen physiologischen Signale (SA, SB, SC, SN) eine zeitliche Beziehung zwischen einer rechtsventrikulären Aktionspotentialaktivität und einer linksventrikulären Aktionspotentialaktivität identifiziert;
wobei das Verarbeitungsmodul (16) so konfiguriert ist, dass es die verschiedenen physiologischen Signale (SA, SB, SC, SN) in verschiedenen Verarbeitungskanälen (163A, 163N) verarbeitet; und
wobei das Verarbeitungsmodul (16) so konfiguriert ist, dass es einen der verschiedenen Verarbeitungskanäle (163A, 163N) zur Überwachung eines bestimmten Wellenformmerkmals (F1-F3) auf der Grundlage einer Identifizierung des bestimmten Wellenformmerkmals (F1-F3) in den verschiedenen physiologischen Signalen (SA, SB, SC, SN) auswählt.

2. Das implantierbare medizinische Gerät (1) nach Anspruch 1, wobei die Anordnung mehr als drei Elektrodenpole (12, 13, 14, 18) enthält.

3. Das implantierbare medizinische Gerät (1) nach Anspruch 1 oder 2, wobei das Verarbeitungsmodul (16) so konfiguriert ist, dass es ein Wellenformmerkmal (F1-F3) in mindestens einem der verschiedenen physiologischen Signale (SA, SB, SC, SN) identifiziert.

4. Das implantierbare medizinische Gerät (1) nach Anspruch 3, wobei das Wellenformmerkmal (F1-F3) ein P-Wellen-Merkmal ist.

5. Das implantierbare medizinische Gerät (1) nach einem der vorstehenden Ansprüche, wobei das Verarbeitungsmodul (16) so konfiguriert ist, dass es mindestens eines der folgenden Merkmale bestimmt: einen P-Wellen-Zeitpunkt (T1), einen P-Wellen-Level (A1), eine P-Wellen-Länge (L1), einen QRS-Wellenform-Zeitpunkt (T2), einen QRS-Wellenform-Level (A2), eine QRS-Wellenform-Länge (L2), einen T-Wellen-Zeitpunkt (T3), einen T-Wellen-Level (A3) und eine T-Wellen-Länge (L3) in mindestens einem der verschiedenen physiologischen Signale (SA, SB, SC, SN).

6. Das implantierbare medizinische Gerät (1) nach einem der vorstehenden Ansprüche, wobei das Verarbeitungsmodul (16) so konfiguriert ist, dass es in mindestens einem der verschiedenen Verarbeitungskanäle (163A, 163N) mindestens eine der folgenden Funktionen ausführt: eine Verstärkung, eine Analog-Digital-Wandlung und eine Filterung.

7. Das implantierbare medizinische Gerät (1) nach einem der vorstehenden Ansprüche, wobei das Verarbeitungsmodul (16) so konfiguriert ist, dass es das erste Signal in dem ersten Verarbeitungskanal und das zweite Signal in dem zweiten Verarbeitungskanal synchron verarbeitet.

8. Das implantierbare medizinische Gerät (1) nach einem der vorstehenden Ansprüche, wobei die Elektrodenpole (12, 13, 14) entlang einer Längsachse (L) ausgerichtet angeordnet sind.

9. Das implantierbare medizinische Gerät (1) nach Anspruch 8, wobei der erste Elektrodenpol (12) und der zweite Elektrodenpol (13) zwischen sich einen ersten Signalempfangsvektor (A) definieren, der entlang der Längsachse (L) zeigt, der zweite Elektrodenpol (13) und der dritte Elektrodenpol (14) zwischen sich einen zweiten Signalempfangsvektor (B) definieren, der entlang der Längsachse (L) zeigt, und der erste Elektrodenpol (12) und der dritte Elektrodenpol (14) zwischen sich einen dritten Signalempfangsvektor (C) definieren, der entlang der Längsachse (L) zeigt.

10. Das implantierbare medizinische Gerät (1) nach einem der vorstehenden Ansprüche, umfassend ein Gehäuse (10), wobei der erste Elektrodenpol (12) an einem ersten Ende des Gehäuses (10) angeordnet ist, der zweite Elektrodenpol (13) an einem dem ersten Ende gegenüberliegenden zweiten Ende des Gehäuses (10) angeordnet ist und der dritte Elektrodenpol (14) an einer Stelle zwischen dem ersten Ende und dem zweiten Ende angeordnet ist.

11. Das implantierbare medizinische Gerät (1) gemäß einem der vorstehenden Ansprüche, wobei das Verarbeitungsmodul (16) so konfiguriert ist, dass es die Herzfunktion auf der Grundlage eines Vergleichs von mindestens zwei der verschiedenen physiologischen Signale (SA, SB, SC, SN) bewertet.

12. Ein Verfahren zum Betreiben eines implantierbaren medizinischen Gerätes (1) zum Erfassen physiologischer Signale, umfassend:
Erfassen physiologischer Signale unter Verwendung einer Anordnung aus mindestens einem ersten Elektrodenpol (12), einem zweiten Elektrodenpol (13) und einem dritten Elektrodenpol (14); und
Verarbeiten von Signalen, die über die Anordnung aus mindestens dem ersten Elektrodenpol (12), dem zweiten Elektrodenpol (13) und dem dritten Elektrodenpol (14) empfangen werden, unter Verwendung eines Verarbeitungsmoduls (16), wobei das Verarbeitungsmodul (16) verschiedene physiologische Signale (SA, SB, SC, SN) verarbeitet, die von verschiedenen Paaren von Elektrodenpolen der Anordnung aus mindestens dem ersten Elektrodenpol (12), dem zweiten Elektrodenpol (13) und dem dritten Elektrodenpol (14) empfangen werden, und auf der Grundlage der Verarbeitung eine Herzfunktion bewertet;
wobei das Verarbeitungsmodul (16) auf der Grundlage der verschiedenen physiologischen Signale (SA, SB, SC, SN) eine zeitliche Beziehung zwischen einer rechtsventrikulären Aktionspotentialaktivität und einer linksventrikulären Aktionspotentialaktivität identifiziert;
wobei das Verarbeitungsmodul (16) die verschiedenen physiologischen Signale (SA, SB, SC, SN) in verschiedenen Verarbeitungskanälen (163A, 163N) verarbeitet; und
wobei das Verarbeitungsmodul (16) einen der verschiedenen Verarbeitungskanäle (163A, 163N) zur Überwachung eines bestimmten Wellenformmerkmals (F1-F3) auf der Grundlage einer Identifizierung des bestimmten Wellenformmerkmals (F1-F3) in den verschiedenen physiologischen Signalen (SA, SB, SC, SN) auswählt.

## Revendications

1. Dispositif médical implantable (1) destiné à détecter des signaux physiologiques, comprenant :
un agencement d'au moins un premier pôle d'électrode (12), un deuxième pôle d'électrode (13) et un troisième pôle d'électrode (14), ledit agencement d'au moins le premier pôle d'électrode (12), le deuxième pôle d'électrode (13) et le troisième pôle d'électrode (14) étant configurés pour détecter des signaux physiologiques ; et
un module de traitement (16) destiné à traiter des signaux physiologiques reçus par l'intermédiaire dudit agencement d'au moins le premier pôle d'électrode (12), le deuxième pôle d'électrode (13) et le troisième pôle d'électrode (14) ;
dans lequel le module de traitement (16) est configuré pour traiter différents signaux physiologiques (SA, SB, SC, SN) reçus par différentes paires de pôles d'électrode de l'agencement d'au moins le premier pôle d'électrode (12), le deuxième pôle d'électrode (13) et le troisième pôle d'électrode (14) et pour évaluer une fonction cardiaque en se basant sur le traitement ;
dans lequel le module de traitement (16) est configuré pour identifier une relation temporelle entre une activité potentielle d'action ventriculaire droite et une activité potentielle d'action ventriculaire gauche en se basant sur les différents signaux physiologiques (SA, SB, SC, SN) ;
dans lequel le module de traitement (16) est configuré pour traiter les différents signaux physiologiques (SA, SB, SC, SN) dans différents canaux de traitement (163A, 163N) ; et
dans lequel le module de traitement (16) est configuré pour sélectionner l'un des différents canaux de traitement (163A, 163N) pour surveiller une caractéristique de forme d'onde (F1-F3) particulière en se basant sur une identification de la caractéristique de forme d'onde (F1-F3) particulière dans les différents signaux physiologiques (SA, SB, SC, SN).

2. Dispositif médical implantable (1) selon la revendication 1, dans lequel l'agencement contient plus de trois pôles d'électrode (12, 13, 14, 18).

3. Dispositif médical implantable (1) selon la revendication 1 ou 2, dans lequel le module de traitement (16) est configuré pour identifier une caractéristique de forme d'onde (F1-F3) dans au moins l'un des différents signaux physiologiques (SA, SB, SC, SN).

4. Dispositif médical implantable (1) selon la revendication 3, dans lequel la caractéristique de forme d'onde (F1-F3) est une caractéristique d'onde P.

5. Dispositif médical implantable (1) selon l'une des revendications précédentes, dans lequel le module de traitement (16) est configuré pour déterminer au moins une parmi une temporalité d'onde P (T1), un niveau d'onde P (A1), une longueur d'onde P (L1), une temporalité de forme d'onde QRS (T2), un niveau de forme d'onde QRS (A2), une longueur de forme d'onde QRS (L2), une temporalité d'onde T (T3), un niveau d'onde T (A3) et une longueur d'onde T (L3) dans au moins l'un des différents signaux physiologiques (SA, SB, SC, SN).

6. Dispositif médical implantable (1) selon l'une des revendications précédentes, dans lequel le module de traitement (16) est configuré pour réaliser, dans au moins l'un des différents canaux de traitement (163A, 163N) au moins un parmi une amplification, une conversion analogique/numérique et un filtrage.

7. Dispositif médical implantable (1) selon l'une des revendications précédentes, dans lequel le module de traitement (16) est configuré pour traiter de manière synchrone ledit premier signal dans ledit premier canal de traitement et ledit second signal dans ledit second canal de traitement.

8. Dispositif médical implantable (1) selon l'une des revendications précédentes, dans lequel les pôles d'électrode (12, 13, 14) sont agencés de manière alignée le long d'un axe longitudinal (L).

9. Dispositif médical implantable (1) selon la revendication 8, dans lequel le premier pôle d'électrode (12) et le deuxième pôle d'électrode (13) définissent un premier vecteur de réception de signal (A) entre eux orienté le long de l'axe longitudinal (L), le deuxième pôle d'électrode (13) et le troisième pôle d'électrode (14) définissent un deuxième vecteur de réception de signal (B) entre eux orienté le long de l'axe longitudinal (L), et le premier pôle d'électrode (12) et le troisième pôle d'électrode (14) définissent un troisième vecteur de réception de signal (C) entre eux orienté le long de l'axe longitudinal (L).

10. Dispositif médical implantable (1) selon l'une des revendications précédentes, comprenant un boîtier (10), dans lequel le premier pôle d'électrode (12) est agencé au niveau d'une première extrémité du boîtier (10), le deuxième pôle d'électrode (13) est agencé au niveau d'une seconde extrémité du boîtier (10) opposée à la première extrémité, et le troisième pôle d'électrode (14) est agencé au niveau d'un emplacement entre ladite première extrémité et ladite seconde extrémité.

11. Dispositif médical implantable (1) selon l'une des revendications précédentes, dans lequel le module de traitement (16) est configuré pour évaluer ladite fonction cardiaque en se basant sur une comparaison d'au moins deux des différents signaux physiologiques (SA, SB, SC, SN).

12. Procédé de fonctionnement d'un dispositif médical implantable (1) destiné à détecter des signaux physiologiques, comprenant les étapes consistant à :
détecter des signaux physiologiques en utilisant un agencement d'au moins un premier pôle d'électrode (12), un deuxième pôle d'électrode (13) et un troisième pôle d'électrode (14); et
traiter, en utilisant un module de traitement (16), des signaux reçus par l'intermédiaire dudit agencement d'au moins le premier pôle d'électrode (12), le deuxième pôle d'électrode (13) et le troisième pôle d'électrode (14), dans lequel le module de traitement (16) traite différents signaux physiologiques (SA, SB, SC, SN) reçus par différentes paires de pôles d'électrode de l'agencement d'au moins le premier pôle d'électrode (12), le deuxième pôle d'électrode (13) et le troisième pôle d'électrode (14) et évalue une fonction cardiaque en se basant sur le traitement ;
dans lequel le module de traitement (16) identifie une relation temporelle entre une activité potentielle d'action ventriculaire droite et une activité potentielle d'action ventriculaire gauche en se basant sur les différents signaux physiologiques (SA, SB, SC, SN) ;
dans lequel le module de traitement (16) traite les différents signaux physiologiques (SA, SB, SC, SN) dans différents canaux de traitement (163A, 163N) ; et
dans lequel le module de traitement (16) sélectionne l'un des différents canaux de traitement (163A, 163N) pour surveiller une caractéristique de forme d'onde (F1-F3) particulière en se basant sur une identification de la caractéristique de forme d'onde (F1-F3) particulière dans les différents signaux physiologiques (SA, SB, SC, SN).
